(19) **Europäisches Patentamt / European Patent Office / Office européen des brevets**

(11) **EP 3 010 581 B1**

(12) **EUROPEAN PATENT SPECIFICATION**

(45) Date of publication and mention
of the grant of the patent:
**11.10.2023 Bulletin 2023/41**

(21) Application number: **14813206.1**

(22) Date of filing: **18.06.2014**

(51) International Patent Classification (IPC):
**A61N 1/05** *(2006.01)*        **A61N 7/00** *(2006.01)*
**A61N 1/372** *(2006.01)*

(52) Cooperative Patent Classification (CPC):
**A61N 1/3787; A61N 1/0551; A61N 1/0558;**
**A61N 1/37205;** A61B 5/283; A61N 1/37211;
A61N 1/37229; A61N 1/3785

(86) International application number:
**PCT/US2014/043023**

(87) International publication number:
**WO 2014/205129 (24.12.2014 Gazette 2014/52)**

(54) **APPARATUS FOR MINIMALLY INVASIVE IMPLANTABLE MODULATORS**

VORRICHTUNG FÜR MINIMALINVASIVE IMPLANTATMODULATOREN

APPAREIL POUR MODULATEURS IMPLANTABLES À INVASION MINIMALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2013 US 201361836544 P**
**18.06.2013 US 201361836536 P**

(43) Date of publication of application:
**27.04.2016 Bulletin 2016/17**

(73) Proprietor: **Nalu Medical, Inc.**
**Carlsbad, CA 92008 (US)**

(72) Inventors:
• **YAKOVLEV, Anatoly**
**Santa Clara, California 95054 (US)**

• **PIVONKA, Daniel**
**Palo Alto, California 94603 (US)**

(74) Representative: **Cooley (UK) LLP**
**22 Bishopsgate**
**London EC2N 4BQ (GB)**

(56) References cited:
EP-A1- 2 155 330         WO-A1-2007/068284
WO-A1-2008/066556       WO-A2-2013/035092
US-A1- 2005 043 765     US-A1- 2006 149 330
US-A1- 2006 224 225     US-A1- 2008 132 981
US-A1- 2008 161 803     US-A1- 2009 204 170
US-A1- 2010 249 888     US-A1- 2011 184 337
US-A1- 2011 301 670     US-A1- 2012 296 329
US-A1- 2013 012 866

## Description

### FIELD

The present description generally relates to apparatus for mechanically modulating nerves inside the body, particularly for long-term use in low power miniaturized implantable devices. The present description also relates to an apparatus for minimally invasive implantable devices for therapeutic and/or diagnostic purposes The invention is set out in the appended claims.

### BACKGROUND

Implantable medical devices are used for a number of different conditions and therapies. Many implantable medical devices are large in size, because they rely on batteries as an energy source. Neuromodulation devices, such as pacemakers, also have long leads. Leads have electrodes at one end, which are placed next to the stimulation site and are connected to the pacemaker, which is implanted separately under the skin. The batteries have to be replaced every several years. These devices require invasive implantation and suffer from poor power efficiency, due to the nature of the electrical stimulation methods. Additionally, these methods gradually become less effective, because the electrical contact with the tissue degrades over time. Also, these methods can potentially cause nerve damage, because of the high voltages and currents that are applied.

There are also known methods for wirelessly communicating data and providing power, particularly from a location exterior to a body and to an implantable device disposed within a body of tissue. One such discussion is provided in the patent application entitled "Method and Apparatus for Efficient Communication with Implantable Devices" filed as U.S. Patent Application Serial No. 13/734,772, on January 4, 2012 (US 2013-0215979).

Nerve stimulation treatments have shown increasing promise, showing potential in the treatment of many chronic diseases, including drug-resistant hypertension, motility disorders in the intestinal system, metabolic disorders arising from diabetes and obesity, and chronic pain conditions among others. Many of these treatments have not been developed effectively, because of the lack of miniaturization and power efficiency, in addition to other factors. Wirelessly powered systems with communication are desirable, because they can be miniaturized and have no need for battery replacements. However, wireless systems have an even more restrictive power budget.

WO2008066556 describes a system comprising an implantable medical device and an actively deployable clip attached to the implantable medical device to restrict movement of the implantable medical device once the clip is deployed within a body of a patient. The clip includes an electrically conductive portion. The implantable medical device may be implanted proximate to any suitable tissue site within the patient, and in one embodiment, the implantable medical device is implanted proximate to an occipital nerve or a trigeminal nerve of the patient.

US2006149330 describes a cardiac rhythm management apparatus including a proximal housing, a distal housing and a lead. The proximal housing includes a first energy storage device. The distal module is implantable within a patient's heart, and includes a second energy storage device, at least one electrode, and a control module. The control module controls the delivery of at least one electrical stimulus from the second energy storage device to a location in communication with the patient's heart. The lead connects the proximal housing to the distal module and is configured to communicate one or more digital signals between the proximal housing and the distal module.

WO2013035092 describes apparatus and methods for use with a blood vessel of a subject, including a stent (20) configured to be placed in the blood vessel. The stent includes at least first, second (34), and third strut portions disposed along the stent. The first and second strut portions are coupled to one another at a first junction that facilitates bending of the first and second strut portions with respect to one another. The second and third strut portions are coupled to one another at a second junction that facilitates bending of the second and third strut portions with respect to one another. At least one electrode is disposed on at least an outer surface of the stent. Other applications are also described.

US2005043765 describes an intravascular implantable pacing and/or defibrillation system. The described system includes a pulse generator that is implantable within a blood vessel and proportioned to blood flow through the blood vessel, and at least one electrode attachable to the pulse generator. During implantation, the pulse generator is introduced into a patient's vasculature, advanced to a desired vessel and anchored in place within the vessel. The electrode or electrodes are placed within the heart or surrounding vessels as needed to deliver electrical pulses to the appropriate location.

US2006224225 describes an intravascular implantable pacing and/or defibrillation system. The described system includes a pulse generator that is implantable within a blood vessel and proportioned to blood flow through the blood vessel, and at least one lead attachable to the pulse generator. During implantation, the pulse generator is introduced into a patient's vasculature, advanced to a desired vessel and anchored in place within the vessel. The lead or leads are placed within the heart or surrounding vessels as needed to deliver electrical pulses to the appropriate location.

US2009204170 describes a wireless electrostimulation system comprising a wireless energy transmission source, and an implantable cardiovascular wireless electrostimulation node. A receiver circuit comprising an inductive antenna can be configured to capture magnetic energy to generate a tissue electrostimulation. A tissue electrostimulation circuit, coupled to the receiver circuit, can be configured to deliver energy captured by the receiver circuit as a tissue electrostimulation waveform. Delivery of tissue electrostimulation can be initiated by a therapy control unit.

US2011301670 describes apparatus and methods for identifying a subject as suffering from polyneuropathy. In response to the identifying, electrodes are placed within 1 mm of a tibial nerve of the subject, the electrodes being disposed on a housing that is at least partially flexible. The electrodes are driven to treat the polyneuropathy by driving a current into the tibial nerve. Other embodiments are also described.

WO2007068284 describes an intra cardiac device. The device comprises means for transforming kinetic energy from heart tissue movement into electrical energy in use, from which electrical energy information in respect of heart function is obtainable. Furthermore, a system is disclosed, comprising one such intra cardiac device and at least one receiver, wherein the intra cardiac device comprises means of communication, through which said at least one device communicates with the receiver(s) wirelessly. In this way energy from heart movement provides self-contained intra cardiac devices for conveniently monitoring or stimulating a patient's heart. EP 2 155 330 and US 2010/249888 describe alternative assemblies.

BRIEF SUMMARY

The present invention is set out in the appended claims. The embodiments, examples or aspects according to the present description which do not fall within the scope of said claims are provided for illustrative purposes only and do not form part of the present invention. In particular, any method described herein is excluded from the scope of the present invention.

In some embodiments the implanted system is constructed and arranged to harvest energy from the environment surrounding the implanted system. The implanted system can be constructed and arranged to harvest energy selected from the group consisting of: heat energy; motion energy; and combinations thereof.

In some embodiments the implanted system further comprises a power receiver constructed and arranged to receive power transmitted transcutaneously to the implanted system. The power receiver can be constructed and arranged to receive power selected from the group consisting of: radiofrequency energy; ultrasound energy; and combinations thereof.

In some embodiments the implanted system further comprises a power supply. The power supply can comprise a component selected from the group consisting of: a battery; a capacitor; and combinations thereof.

In some embodiments the tissue modulator comprises a mechanical effector constructed and arranged to physically engage a nerve and an electromagnetic actuator coupled to the mechanical effector, and the electromagnetic actuator is constructed and arranged to induce motion, induce rotation, change a dimension, and/or change a shape of the mechanical effector in a way that would modulate the nerve.

In some embodiments the tissue modulator comprises at least one actuator constructed and arranged to deliver the mechanical forces to tissue and/or modulate a nerve. The actuator can comprise a piezoelectric based actuator. The actuator can comprise a thermal actuator. The actuator can comprise a motor. The actuator can comprise a linear actuator. The actuator can comprise a vibrating beam. The actuator can be controlled by varying a parameter of the applied force selected from the group consisting of: amplitude; frequency; duty cycle; duration; and combinations thereof. The actuator can be controlled by varying a magnetic field. The actuator can be controlled by varying current.

In some embodiments the apparatus further comprises an external system. The external system can be configured to control the implanted system and/or the tissue modulator. The external system can be configured to transmit data to the implanted system and/or the tissue modulator. The external system is configured to transmit power transcutancously to the implanted system and/or the tissue modulator. The power transmitcr is constructed and arranged to transmit power selected from the group consisting of: radiofrequency energy; ultrasound energy; and combination thereof. The external system can be configured to magnetically couple to the implanted system and/or the tissue modulator. The magnetic coupling can be configured to cause movement of the tissue modulator to induce the physiological response from tissue.

According to another aspect of the present disclosure an therapeutic and/or diagnostic implant for inducing a physiologic response in tissue and/or monitoring tissue activity comprises a body portion for implanting with a patient and at least one extension attached to the body portion. The extension is constructed and arranged to transition from a compacted state to an expanded state after the body portion is implanted in the patient.

In some embodiments the therapeutic and/or diagnostic implant is constructed and arranged to perform a function selected from the group consisting of: delivering energy to tissue; delivering therapy to tissue; delivering pharmaceutical agent to tissue; modulating tissue; sensing physiological and/or neural activity; sensing environmental conditions; sensing therapeutic outcomes and/or delivered therapy parameters; and combinations thereof.

In some embodiments the implant is constructed and arranged to be delivered through an introducer. The introducer can comprise a needle. The introducer can be less than 5mm, 4mm, 3mm, 2mm, or 1mm in diameter. The introducer can comprise a flexible cannula and a removable rigid cannula slidingly received by the flexible cannula.

In some embodiments transitioning from the compacted state to the expanded state comprises a physical change selected from the group consisting of: unfolding; unrolling; unfurling; and combinations thereof.

In some embodiments the extension comprises an energy harvesting element.

In some embodiments the extension comprises an electrode.

In some embodiments the extension comprises an energy storage element.

In some embodiments the extension comprises two sliding beams. The extension can comprise a cuff electrode, and the compacted state comprises a relatively straight configuration and the expanded state comprises a ring configuration.

In some embodiments the extension is constructed and arranged to be detachable from the body portion.

In some embodiments the extension comprises an inflatable portion constructed and arranged to expand when filled with fluid. The fluid can comprise a material selected from the group consisting of: a gas; a liquid; a gel; and combinations thereof. In some embodiments the fluid may be liquid, such as saline.

In some embodiments the extension comprises an anchor element. The anchor element can comprise an element selected from the group consisting of: threads; arrow-shaped element; harpoon-shaped element; and combinations thereof. The anchor element can comprise threads. The therapeutic and/or diagnostic implant can further comprise an introducer for delivering the body portion within the patient.

BRIEF DESCRIPTION OF DRAWINGS

Fig. 1 illustrates an apparatus comprising an implanted system wirelessly coupled with a system external to the body;
Fig. 2 illustrates a diagram of an active implanted system ;
Fig. 3 illustrates a minimally invasive implantable system delivered through a needle ;
Fig. 4 illustrates a fully enclosed implant encapsulated to be delivered with minimally invasive methods;
Fig. 5 illustrates a minimally invasive implantable system with an unfolding extension after implantation;
Fig. 6 illustrates several embodiments of a minimally invasive device with unfolding or expanding extensions;
Fig. 7 illustrates a minimally invasive device with expandable electrodes ;
Fig. 8 illustrates a minimally invasive device with harpoon or barbed electrodes;
Fig. 9 illustrates a minimally invasive device with flexible electrodes that have barbed or harpoon tips for attaching to tissue ;
Fig. 10 illustrates a minimally invasive device with a screw electrode for attaching to tissue;
Fig. 11 illustrates a minimally invasive device with a conformable attachment to a target tissue site, and the mechanism for this attachment;
Fig. 12 illustrates an implementation of a mechanical modulator with an external magnetic field source/generator and an implanted magnetic material or implanted current-carrying wires;
Fig. 13 illustrates an implementation of a mechanical modulator with an external electromagnet creating fields that influence an implanted magnetic material or current-carrying wires;
Fig. 14 illustrates a mechanical actuator using an electromagnet to create mechanical forces for modulating tissues ;
Fig. 15 illustrates wire and current flow characteristics that experience forces or torques in the presence of a magnetic field;
Fig. 16 illustrates a piezoelectric actuator for mechanically modulating tissues .

DETAILED DESCRIPTION

Various examples described herein include a stimulation apparatus that includes an implantable portion, such as an implantable portion configured to stimulate tissue via mechanical actuation. Some aspects include needle-injectable implants, such as needle injectable implants that comprise expandable (e.g. unfolding and/or unrolling) extensions. This disclosure also describes methods of making and using apparatus for needle injectable implantable devices for therapeutic and/or diagnostic purposes, which can also be capable of conventional electrical modulation or new types of mechanical modulation of tissues.

Also described herein are methods of making and using an apparatus for minimally invasive implantable devices for therapeutic and/or diagnostic purposes. A small form-factor for implants would allow for implantation surgery to be minimally invasive that can potentially be performed with a doctor's office procedure as opposed to a surgery. The small form factor can be achieved by packaging these devices into a shape that can be injected directly into the desired location within a body of tissue through a needle using an injection device similar to a syringe.

The needle injectable implant can be packaged in a sterile needle and packaged together with a disposable injection device. The needle and the injection device can be similar to a conventional needle and syringe and can be customized for a particular therapy application.

The injectable device may include a main body, which is similar in shape to the injection needle. The device may also include one or more optional extensions. Optional extensions can be antenna, electrodes, leads, or other extensions that also fit in the injection device and needle. These extensions are implanted together with the main body or can be delivered separately and assembled inside the body. Furthermore, the extensions can be made flexible to conform to anatomy. The optional extension would allow for the stimulation device and electrodes to be at a different depth from the tissue surface than the antenna, for example. In that case, the antenna may be positioned close to the tissue surface. This would allow for less tissue absorption when delivering energy to, or communicating with, the implant and thus improve link gain between the external antenna and the implanted device antenna.

The main body and the extensions can also be compacted in the needle and unfold, unroll and/or unfurl (hereinafter "unfold") or otherwise expand (i.e. transition from a compacted state to an expanded state) in order to obtain a different shape after the implant is ejected from the needle. One reason for this expansion can be to make the antenna radar cross section (RCS) larger in order to capture more energy. This can be beneficial for deeper implants by enabling the antenna to harvest more energy and have better link gain. Another reason for expandable extension can be to position stimulation or sensing electrodes further apart. The expandable extensions and/or device are advantageous because they allow for use of smaller diameter needles and thus less invasive implantation procedure while still allowing for optimization of the device design.

Various aspects also provide apparatus and methods for mechanical devices that modulate nerves with minimal power requirements. These devices can be miniaturized to millimeter and sub-millimeter scales, depending on the needs of the application. Additionally, performance does not degrade in the same way as electrical stimulation, because electrical contacts with tissue are not required. This allows these devices to be used with more sensitive nerves and tissues without causing damage. Also, because of the much lower voltage and current requirements, these methods are well-suited for fully wireless implants.

Mechanical forces can be generated from a wide variety of sources, including magnetic forces, electromagnetic forces, piezoelectric forces, or thermal expansion forces among others. These forces have different scaling properties and are most effective at different sizes. For maximum miniaturization and power efficiency, electromagnetic forces have significant potential for nerve stimulation. There are several examples that implement these forces. One implementation uses electric currents to create a magnetic field, which then manipulates either a permanent magnet or magnetic material. This method is similar to a miniaturized solenoid and can generate fairly significant forces. A variation of this method is to generate the magnetic field externally with either a magnet or an electromagnet so that the implanted device is entirely passive. In another implementation, a magnetic field is established and the mechanical force is generated from forces on current-carrying wires, which are very simple to construct and can be very small. Again, the magnetic field can be generated either internally or externally with either a magnet or electromagnet. Both implementations have flexibility in their construction and operation, which can be adapted to specific therapies.

In some implementations, magnetic fields can be generated externally, to allow either passive or active devices to create mechanical force. The actuators can be interfaced with known remotely powered devices, battery powered devices, or passive setups; alternatively, they can be incorporated in the design of new devices.

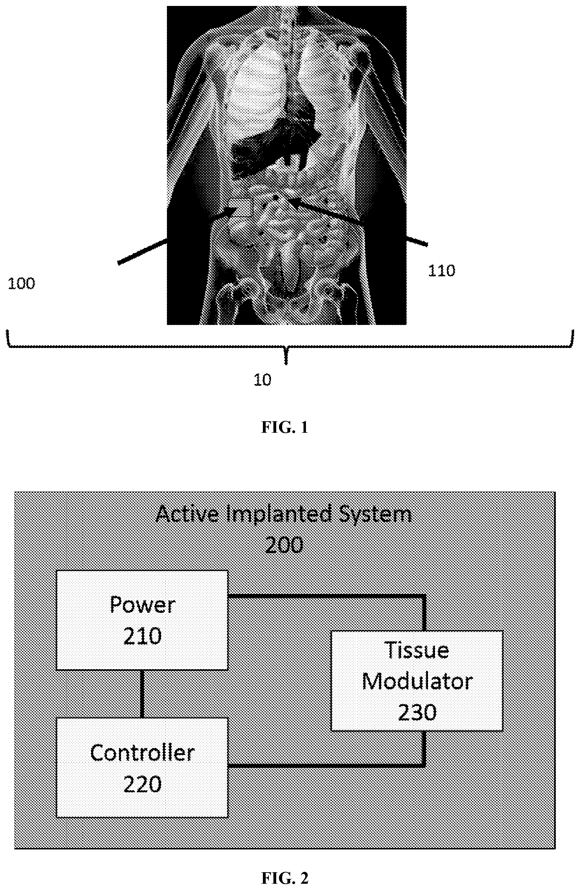

For certain therapies, other mechanical methods may have advantages over electromagnetic forces because they have no need for a magnetic field. Typically, these other methods will be larger, with more limitations under the operating conditions, though may offer advantages in the magnitude of the force or they may be better suited to the environment. Piezoelectric methods can take the form of vibrating beams or motors, and can generate significant forces at specific frequencies. Thermoelectric actuators may also be useful, because they operate simply at small sizes. Implantable systems are limited in their design by the power budget, which restricts both miniaturization and functionality. Examples described herein include a minimally invasive implantable system with an external system . FIG. 1 depicts apparatus 10 configured to stimulate tissue and comprising implantable system 110 and external system 100. Depending on the application, this implant can be placed in different locations inside the body and can include a variety of systems. This implant can be either active or passive (e.g. include one or more active or passive components, respectfully), and the external equipment can consist of power transmission, data transmission, and/or magnetic field generation. Also, depending on the application, there may be one or more implantable systems 110 coupled with a single or multiple external systems 100.

A diagram of another tissue stimulating active implant system 200, according to another implementation, is shown in FIG. 2. Active implant 200 can comprise a power source 210, which can include a power receiver and/or a power supply. The power source 210 can be either locally powered such as with batteries, capacitors, etc, and/or by harvesting energy from the body (heat, motion and/or chemical energy, for example) and/or remotely powered, such as with RF energy (near-, far-, or mid-field powering) or ultrasound. The controller 220 can be designed to work with existing devices, and can allow for either analog or digital control of the modulator, power systems, or other components. Controller 220 can also manage incoming data and communications with other systems. Data for controlling the stimulation profile can be received from an external transmitter (e.g. external system 100 of FIG. 1). Implanted system 200 can comprise a tissue modulator 230, which can be surrounded by a housing (e.g. a sealing housing) surrounding at least a portion of tissue modulator 230. The modulator 230 can employ any of the active methods that are described herein, including conventional electrical methods and new methods for mechanical modulation as described herein. Modulator 230 may include an electromagnetic transducer such as a tranducer through which current flows, to generate a constant and/or varying magnetic field. Modulator 200 may include a magnetic transducer, such as a transducer comprising a passive magnet and/or an electromagnet. Modulator 230 may include an actuator that delivers mechanical force to tissue, such as an actuator selected from the group consisting of: a piezoelectric-based actuator; a thermal actuator; a motor; a linear actuator; a vibrating beam; and combinations of these. Modulator 230 may include an actuator that is controlled by varying one or more of: amplitude; frequency; duty cycle; and duration. Modulator 230 can be controlled by varying a magnetic field and/or by varying current.

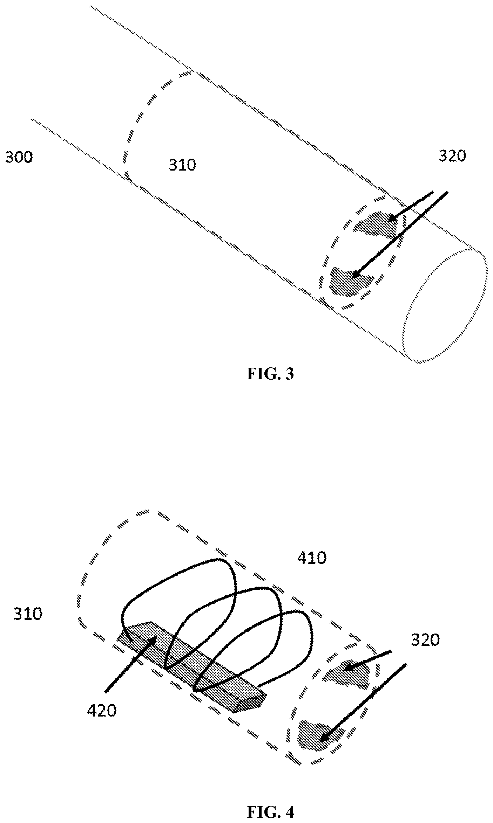

In various implementations, the implant portion of the system may perform any of a number of desired goals of diagnosis, therapy, or both. A major advantage of the implants described herein is that they are miniaturized and packaged such that they can be easily implanted using minimally invasive methods, such as via injection through a needle, for example, without the need for surgery, as shown in FIGS. 3 and 4. The implant main body 310 can fit inside of a delivery needle or introducer 300. The implant main body 310 can also include one or more electrodes 320 that interface with the tissue or environment for modulation or sensing. The implant body 310 can also contain one or more fully enclosed antennas 410 and one or more integrated circuits 420 and other discrete passive or active components. The electrodes 320 can again interface with the tissue or environment.

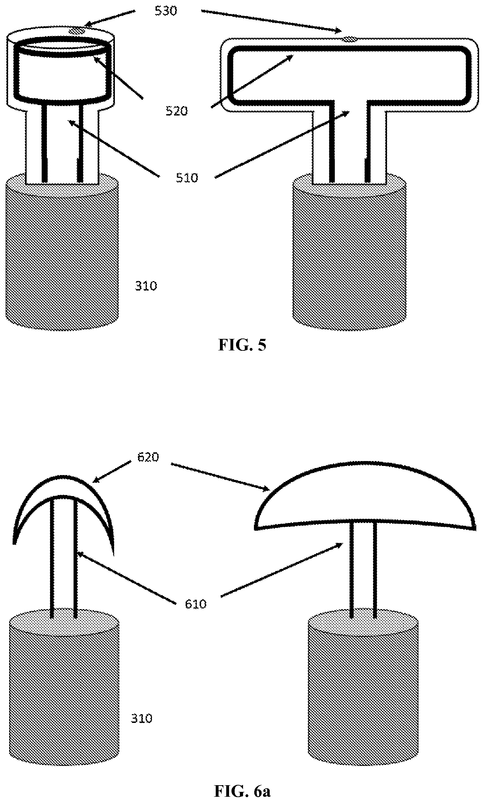

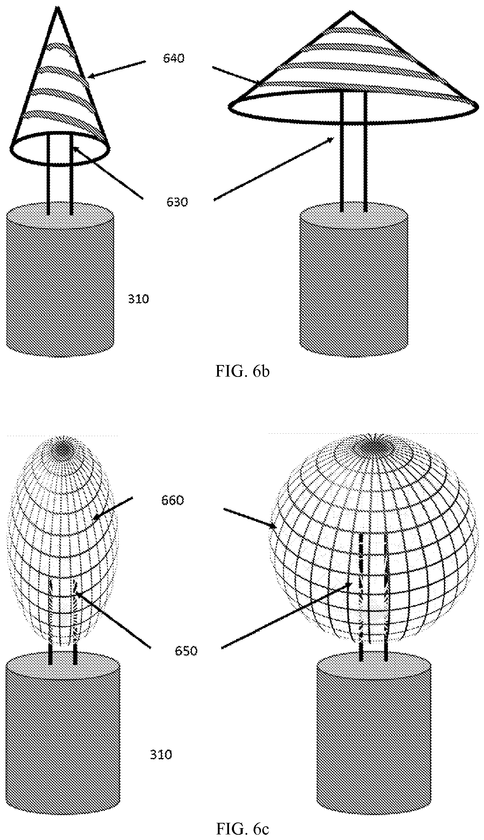

The device itself can be completely contained in a non-deformable package or contain a rigid body with one or more extensions similar to 510 that can be made unfoldable, expandable, or detachable for form-factor or shape adaptation during or after the implantation procedure as shown in FIG. 5 and FIG. 6. Methods for delivering different types of unfolding or expanding extensions are illustrated in 510, 610, 630, and 650.

In these figures, the flexible extensions 510, 610, 630, and 650 are illustrated in compact form on the left side and in expanded form on the right side of the figures. Some embodiments may include a rolled up extension which forms a rectangular antenna substrate 510, a parachute-like structure 610, an umbrella-like structure 630, and/or a spherical antenna structure 650. These structures may include printed conductor traces 520, 620, 640, and 660, which may form one or more planar loop, spiral, or dipole antennas when unfolded or inflated after implantation. For very small device sizes, the delivered power to the device can be very limited, especially for deeply implanted devices. Therefore, it may be beneficial to implement the antenna on a flexible substrate that can be folded (or rolled) in a way to make the device injectable with a needle. Examples of unfolding or expanding antennas are shown in 520, 620, 640, and 660. After the injection, the antenna can be made to unfold (or unroll) such as to make its radar cross section (RCS) larger and thus increase the amount of power it can harvest. Additionally, the antenna only or antenna together with the energy harvesting circuitry (such as matching network and rectifier) can be made separate from the rest of the implant in a form of unfolding extension. This would allow for the antenna to be closer to the tissue surface and therefore would reduce separation distance between the external transmitter and the implant antenna and would therefore potentially improve the link gain. Having rectification circuitry close to the antenna can be advantageous to maintain reasonably high quality factor that is not limited by the transmission line losses. Alternatively, the transmission line can serve as part of the matching network. Some sketches for the implantation of various needle injectable devices are shown in FIG.5 and FIG.6.

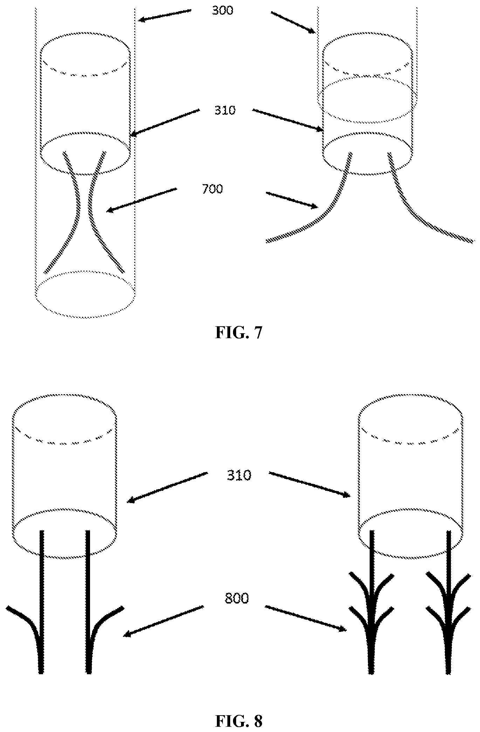

The implant main body 310, together with any of a variety of antennas (a few examples are 520, 620, 640, or 660), may be positioned close to the surface of the tissue by first securing the electrodes at the desired location and then ejecting the device from the needle while the needle is being pulled back. By having electrodes attached to the main body 310 using flexible wires or leads 700, the main body 310 can stay in the needle or injection device 300 while the wires or leads 700 are unraveling as the needle is being pulled back. This is illustrated in FIG. 7. Then at the desired position, the remaining part of the implantable device can be ejected from the needle.

The antenna can be unfolded or unrolled using inflation (e.g. via a fluid such as a gas, a liquid and/or a gel) and/or by incorporating spring-like structures into the antenna material, such that it naturally expands into the usable shape after it is ejected from the needle. In case of inflatable antenna, the needle and the injection mechanism can have a small inflation tube 530 that is connected to the flexible antenna substrate. Once the implant with the folded antenna is ejected from the needle, the inflation mechanism engages, thus inflating the flexible substrate antenna and thereby unfolding it into a usable shape. Once unfolded, the excess inflation gas or liquid (such as saline) can be optionally evacuated, and the inflation tube can be severed from the antenna.

Electrodes 320 can be connected to the main body 310 as simple extensions and can further be made expandable after ejection from the needle. This can benefit the electrode configuration and placement, which can be adapted based on the needs of the application. The electrodes can be made from semi-rigid material and be fabricated such that they expand like a spring, tweezer-like element, or other expandable structure 700. Because of their flexibility, when placed inside the needle, they would naturally be compressed and therefore easily delivered with a desired separation. This process results in a larger separation than needle otherwise allows as shown in FIG. 7.

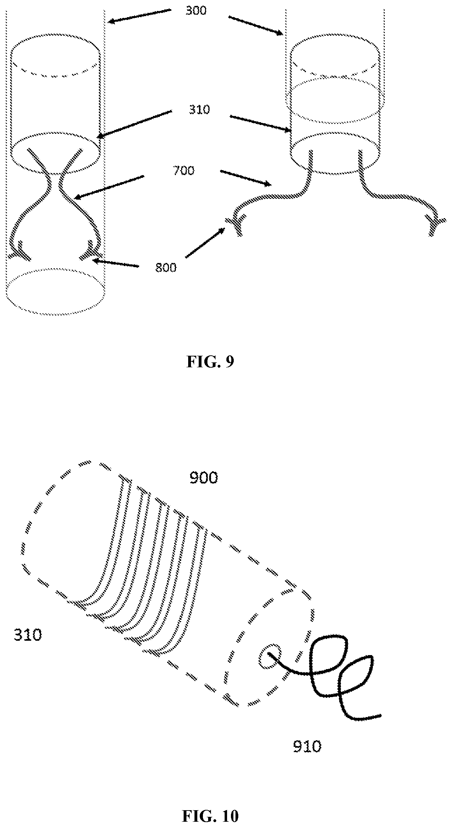

It may be beneficial to fix the device in position to prevent it from moving around and potentially for better electrode/tissue contact for some implantable devices. In this case, electrodes can include an anchor element 800, and can include a harpoon-shaped element 800; an arrow-shaped element 800; and/or threads, as shown in FIG. 8 and FIG. 9. The barbed electrodes 800 puncture and attach to tissues and fix the device in place. These configurations would penetrate the desired tissue or organ and prevent it from being detached from it. The barbed electrodes 800 can also be used at the tip of the flexible electrodes 700 that expand upon delivery for better or more precise placement of the interface. This also allows for a simpler implantation procedure without any stitching or otherwise more complex attachment procedure.

Another possible attachment that allows for easy implantation is by incorporating a screw-like electrode on the device as shown in FIG. 10. The screw electrode 910 screws into tissue to fix it in place. The screwing motion can be accomplished with threading 900 on the implant body 310 that has a matching thread on the delivery needle 300. As the implant body 310 is pushed out of the needle, it will rotate due to the threading. The device can then screw into tissue for a better electrode-tissue interface and a secure attachment. Implant body 310 can contain a counter-electrode of opposite polarity to the active electrode 910 when electrode is being used to modulate tissue. Alternatively, the self-attaching structures (800 and 910) can simply be used to fixate the implant 310 to the target tissue.

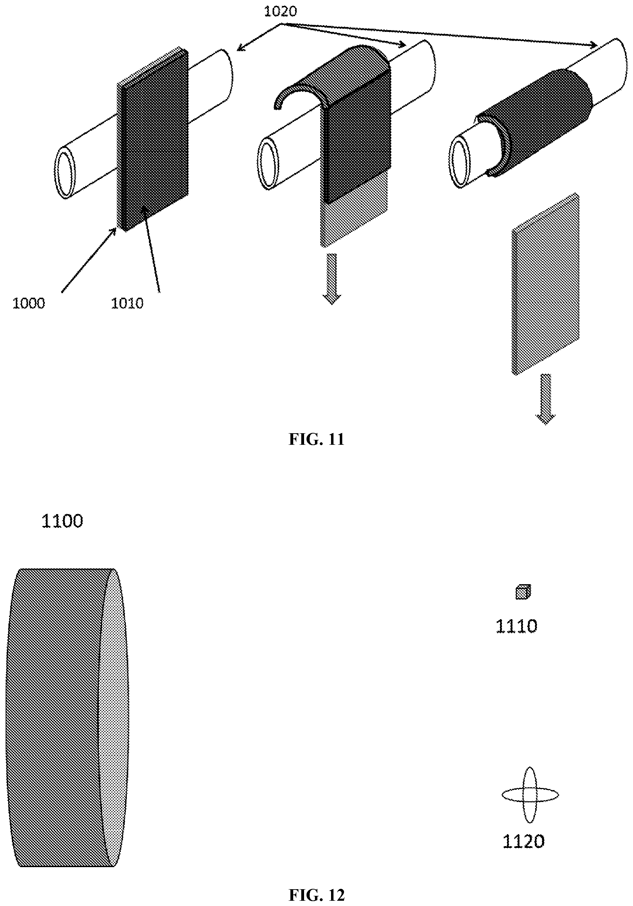

In some other procedures or applications it may be necessary to fix the implantable device around an organ or tissue, such as a vein or an artery. This can be accomplished by making the entire implantable device or some part of it cuff around that organ as shown in FIG. 11. This figure shows a rigid surface 1000 and a bendable or pre-formed substrate 1010. The mechanism for wrapping this system around the target site 1020 is shown. This can be accomplished in several different ways, such as two sliding beams, with one beam (rigid beam 1000) staying in the delivery system 300 and the other (compliant beam 1010) bending around the site 1020 that needs to be treated or diagnosed. As the comformable beam 1010 is ejected from the introducer 300, the rigid beam 1000 separates from the comfortable beam 1010 and the comfortable beam 1010 curves, naturally wrapping around target organ 1020. Another way is to compress a curved substrate 1010 into the needle that would serve as the rigid surface 1000, which would remain straight until ejected, and then would curve into a tight "ring" around the vein or artery as shown in FIG. 11. This method of action would be substantially similar to a slap (snap) bracelet ("Slap Wrap"). This method would contain a bi-stable spring which can be straightened out for delivery. The straightened bracelet cuff could then wrap around the target tissue 1020, forming a tight ring. This is caused by having the bi-stable spring transition into the second stable state (curved state), securing the implant 110 at the target site 320.

In one implementation, the injection apparatus can consist of the main body 310 and a needle or introducer 300. The needle can be made rigid, flexible, or a combination to accommodate a particular implantation procedure. If it may be necessary to navigate around certain areas or organs, the bending or flexible needle can navigate around them and thus avoid certain locations within the body of tissue. This may also be beneficial if the implantable device needs to be positioned at a particular angle that is preferential over a straight line.

In one implementation, an implantation procedure may involve: 1) injection of the needle or introducer 300 and its navigation to the implantation site; 2) initial ejection of the device 110 from the needle 300 until the electrodes are in contact with the stimulation site; 3a) if the device is fully contained without any extensions, then complete the delivery process of the device; 3b) if the device has one or more extensions, then proceed with retraction of the needle where the extension or the leads are unwinding at the same rate while the needle is being extracted (pulled back) from the implantation site; 4) if unfolding or unrolling of the extensions is necessary, proceed with the unfolding procedure once the unfolding extension (510, 610, 630, 650) is ejected from the needle; 5) once the device and all of the optional extensions are fully ejected, remove the needle or introducer 300.

The injection device can further be used for extraction of the implant, if there may be a need. The needle or introducer 300 can be inserted and positioned to come in contact with the device and attach to it with suction forces. The extraction device can be used to extract the entire implant or a part of it, such as a particular extension. This can be done to replace or remove certain parts that can degrade or become unnecessary over time. In that case, the extensions can be made detachable if need be. Alternatively, there may be a temporary (dissolvable) or permanent thread attached to the device, which can extend to the outside the body or be left close to the skin surface in the subcutaneous surface. This way, the device can be removed easily by pulling on the thread.

Both injection and extraction of the implantable device can be guided using some sort of existing imaging modality from outside of the body of tissue, such as ultrasound, X-Ray, MRI, etc. Alternatively, it can be guided by optical fiber or a camera that can be placed at the tip of the injection needle. Furthermore, a technique similar to laparoscopy can be used for the implantation of the device. Conventional guidance methods such as catheters or non-invasive procedures may also be used.

The implant can also be covered with biocompatible, bio-dissolvable, biodegradable materials. In some embodiments, the electrodes may be encapsulated with a bio-dissolvable compound, which is present for ease of implantation, but is dissolved over a period of time post injection. Additionally or alternatively, the electrodes may be coated with drug eluting materials for proper adaptation and to minimize infection, inflammation, and rejection risks.

The needle injectable implant can be packaged in a sterile needle and packaged together with a disposable injection device. The needle and the injection device may be similar to currently available needles and syringes and can be customized for a particular therapy application.

Neuromodulation devices can require significant power to provide therapy, because of the relatively high voltage and current requirements needed to drive stimulation. Essentially, mechanical neuromodulation provides a more efficient conversion from electrical energy to therapeutic stimulation, and offers additional advantages in terms of safety and long-term use. Mechanically activating nerves can be accomplished with very small devices and with power-efficient mechanisms for generating force. Using electromagnetic forces allows for controllable stimulation with a fraction of the voltage and current requirements of electrical stimulation, and requires no direct electrical connection with tissue. This allows the device to be entirely encapsulated in bio-compatible materials if needed, and can therefore operate safely for extended periods of time. Depending on the needs of the application, this actuation can be accomplished by generating a magnetic field to control a magnetic material, by controlling currents in a pre-existing magnetic field, or with some combination of the two. The force from these mechanical actuators can be applied directly to nerves or other tissues to activate them with similar characteristics as electrical stimulation, which results in similar therapeutic outcomes. This mechanical modulation system can be incorporated in the implant body 310 or it can be a separate extension as described. It can also be used in combination with sensors or other extensions, and it may be advantageous to have mechanical modulation operating in conjunction with electrical modulation.

As described, electromagnetic forces can be used in two different ways. The first method manipulates a magnet or a magnetic material by adjusting the magnetic field. This field can be adjusted with permanent magnets or electromagnets. The force on the material is given by

$$F_1 = V_m(M \cdot \nabla)B$$

where $F_1$ is the force, $V_m$ is the volume of the material, $M$ is the magnetization of the material, and $B$ is the strength of the magnetic field. These forces are proportional the gradient of the field and the volume of the structure, though can be quite large even at small sizes. The second force is exerted on currents flowing in the presence of a magnetic field, and is described by the Lorentz force which is

$$F_2 = I(L \times B)$$

where $F_2$ is the force, $I$ is the current flowing through the wire, $L$ is the length of the wire, and $B$ is the magnetic field strength. With strong magnetic fields, large forces can be generated with relatively low currents, making this force very power efficient. This force can be amplified with additional loops of wire. The magnetic field can be generated with permanent magnets or electromagnets, and be either external or internal to the body.

To control the motion of a magnetic material, an electromagnet can be used to adjust the strength of the magnetic field. An effective way of accomplishing this is with several loops of wire around either a magnet or a magnetic material, similar to a solenoid. Current flows through the coil, inducing a magnetic field which applies a force to the magnet or magnetic material inside. This current can be oscillated to produce oscillating forces. The magnetic field generated in the center of the coil by this device is

$$B = \mu_0(N/L)I$$

where $B$ is the magnetic field, $\mu_0$ is the magnetic permeability, $N$ is the number of loops in the coil, $L$ is the length of the coil, and $I$ is the current flowing through the coil. The force on the magnetic material is given by $F_1$ and depends on the size of the material and its magnetization. Ferrite can have relative permeability ~1000, and mumetals can have relative permeability of ~50,000 or more. Using a material with a high relative permeability allows for mm-sized and sub-mm devices to exert large enough forces to activate nerves. These devices can be electrically powered from an implantable device using 10-100x less power than direct electrical stimulation. The magnetic material in this method does not need to be located in the center of the coil, it only needs to be in range of the generated magnetic field to experience a force. Additionally, the restoring force can be supplied mechanically such as with a spring so that the electromechanical force is only exerted in one direction. This is especially useful if a magnetic material is used because the force is insensitive to the direction of the field, it is only sensitive to the gradient.

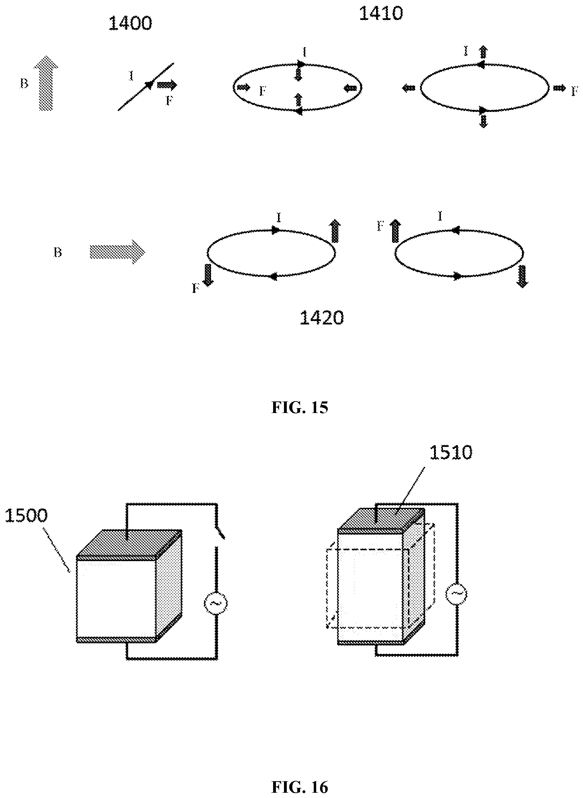

An alternative method employs the forces exerted on current-carrying wires to actuate nerves. This method requires a magnetic field to be present, which can be provided from either a permanent magnet or an electromagnet, which can be either external or internal to the body. The force experienced by the wires is given by $F_2$ and is proportional to the magnetic field and the current. Additional wires can also be used to magnify the force. The current flowing in the wires can be controlled to precisely control the force on them, and several arrangements of wires can surround the nerves to apply a variety of forces to best activate the target nerve. These forces can squeeze, expand, push, or pull the nerve as needed. Several possible arrangements are shown in FIG. 15. This figure shows wires that generate linear forces 1400, loops that experience compression or expansion forces 1410, and loops that experience torques 1420. These arrangements show how several types of forces and torques can be generated. A multitude of wire arrangements are possible, and the actuator can be designed for the targeted nerve with one or more of them. The current in the wires can oscillate with controlled parameters, such as frequency, amplitude, and duty cycle, to induce the desired mechanical forces.

An example of an implanted material experiencing forces due to $F_1$ and $F_2$ is shown in FIG. 12. The magnetic field source/generator 1100 is outside of the body and induces forces on the implanted magnetic material 1110 or on current carrying wire arrangements 1120 through the use of magnetic fields. These forces can cause vibrations, rotations, or linear motion of the implanted material. If the material is attached or in the vicinity of targeted tissues, it can mechanically modulate them.

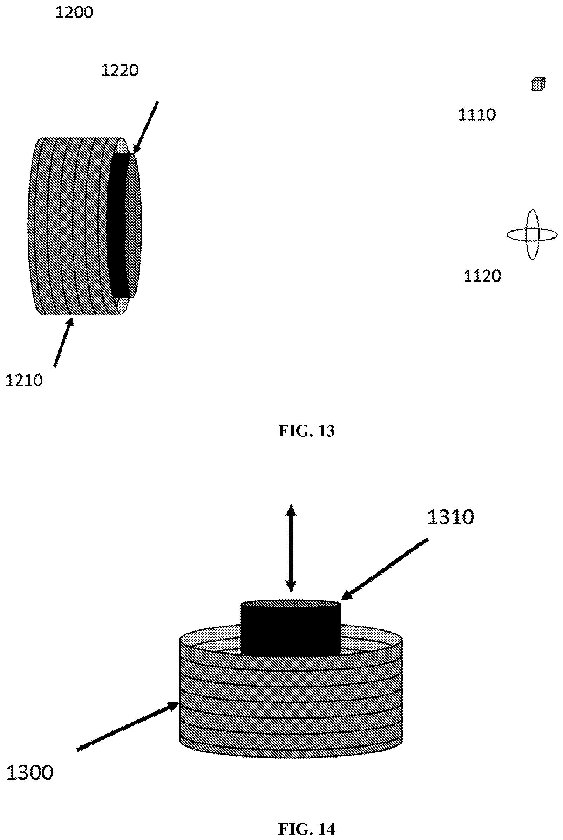

An example of an implanted material experiencing forces due to $F_1$ and $F_2$ using magnetic fields from an electromagnet is shown in FIG. 13. The electromagnet 1200 is outside of the body and induces forces on the implanted magnetic material 1110 or on current carrying wire arrangements 1120 through the use of magnetic fields. The electromagnet 1200 includes coils of wire 1210 and an optional magnetic structure 1220 to enhance the generated field. These forces can cause vibrations, rotations, or linear motion of the implanted material 1110. If the material is attached or in the vicinity of targeted tissues, it can mechanically modulate them.

Alternatively, the implant main body 310 or an extension may include a miniaturized electromagnet, as depicted in FIG. 14. This electromagnet again consists of a smaller arrangement of coils of wire 1300 and a movable magnetic structure 1310. The field caused by currents flowing in the coils of wire 1300 induces forces on the magnetic structure 1310, causing it to move. The currents can be controlled to create controllable motion. These forces can cause vibrations that modulate surrounding tissues mechanically.

For some applications, it may be desirable to generate magnetic fields externally to reduce the complexity of the actuators, to allow for MRI-compatibility if permanent magnets are required, or for miniaturization. This can be accomplished with either an electromagnet or a permanent magnet. This field generation can be co-designed with a power and data transmitter if necessary or can be a separate component of the system. These magnetic fields are generated in the proper direction for the desired force, and higher field strengths result in improved power efficiency, as is evident from the force equations. Electromagnets can be enhanced with high permeability materials, which magnify the field strength.

For either mechanical actuation method, the forces must be designed to stimulate the nerves in a therapeutic way. A key advantage of electromagnetic methods is the ability to operate at a variety of frequencies and to have a highly adjustable force that operates effectively as the device is miniaturized. The stimulation can be optimized in terms of frequency, force, and duty cycle, simply by adjusting the flowing currents. For instance, some nerves are best stimulated with short bursts of high-frequency vibrations, while other nerves are best stimulated with one strong, high force pulse. In the case of fully wireless implants including battery-free, remotely powered devices, this means that the stimulation characteristics can be completely controlled externally with commands from the transmitter and have significant flexibility in operation. Additionally, the scalability of the forces allows for mm and sub-mm operation, allowing for use with devices that can be injected with a needle, placed with an endoscope, or placed with a catheter.

The actual size of the actuators is determined by the needs of the application, as larger nerves may require more force for controlled stimulation. Some nerves, such as baroreceptors, are sensitive to the stretching of blood vessels, and so would require a mechanical structure to apply this type of force. Other nerves can be excited by localized tapping with certain parameters, and would require a different mechanical structure. The arrangements shown in FIG. 15 depict how these types of forces can be accomplished with the described actuators. From the force equations, it is clear that the described force actuation mechanisms have the power efficiency and scalability to meet the needs of these different applications, and the versatility to apply a variety of different forces at different sizes.

Because the forces are easily controlled by manipulating flowing currents, these mechanical methods are easily integrated into existing devices, including fully wireless devices. They can also be implemented on a passive device, where the actuation is due to the manipulation of a magnetic structure with externally applied magnetic fields. For powered devices, the interface circuitry can be designed so that the stimulator controller is either analog or digital. Digital control offers simplicity and more universal operation, though analog control could be better optimized for increased power efficiency. The mechanical stimulators can operate with similar waveforms to that of electrical stimulators, and can therefore serve as a replacement with minimal alteration to existing devices. Additionally, these mechanical techniques can have significant advantages in terms of power efficiency and long-term safety because the tissue impedance does not impact performance and no electrical connection is required. Their high power-efficiency allows for use with remotely powered devices, which have a very limited power budget. This power efficiency would also prolong battery life for conventional devices. The level of miniaturization and efficiency also allows for entirely new devices which may be small enough for non-invasive implantation as previously discussed.

When implanted in the body, these mechanisms can be packaged with epoxies or plastics that encapsulate it for bio-compatibility. These epoxies are ready available, and similar materials can be used to that of existing medical devices. Because no electrical connection is required, the device can be fully encapsulated and protected from the body without hindering performance. The forces will be experienced by the actuators regardless of the surrounding material, as common encapsulating materials are effectively transparent to electric and magnetic fields.

For some therapies, other types of mechanical actuators may be better suited for activating nerves. Piezoelectric materials, such as those shown in FIG. 16, can apply large forces if they are needed, and can constructed in the form of vibrating beams, motors, linear actuators, or other vibrating structures designed with specific resonances. They operate when the piezoelectric 1500 has a voltage applied through an electrical contact 1510, thus inducing mechanical deformation. These devices can be efficient when operating at resonance, and can apply large forces though the forces get fairly weak as they are scaled down to mm-scales. Additionally, thermoelectric actuators could be useful for small devices and would not require the presence of a magnetic field.

## Claims

1. A medical apparatus for interfacing with tissue of a patient comprising:

   an implantable system comprising

   a body portion (310) for implanting within a patient,
   an energy harvesting element or antenna, and
   energy harvesting circuitry,
   wherein the energy harvesting element or antenna and the energy harvesting circuitry are in the form of an extension (510) attached to the body portion;

   an external system (100) that transcutaneously transmits at least one of power or data to the implantable system during operation; and
   an introducer (300) for delivering the body portion (310) and the at least one extension (510) into the patient; wherein the implantable system is constructed and arranged to be delivered through the introducer; and
   wherein the extension (510) is configured to transition from a compacted state to an expanded state to make a radar cross section of the energy harvesting element or antenna larger to increase the amount of power said energy harvesting element or antenna can harvest.

2. The medical apparatus according to claim 1, wherein the medical apparatus is configured to induce a physiologic response in tissue.

3. The medical apparatus according to claim 1 or 2, wherein the medical apparatus is configured to monitor tissue activity.

4. The medical apparatus according to according to any preceding claim, wherein the external system (100) is configured to transcutaneously transmit both power and data to the implantable system during operation.

5. The medical apparatus according to any preceding claim, wherein the implantable system performs a function selected from the group consisting of: delivering energy to tissue; delivering therapy to tissue; delivering pharmaceutical agent to tissue; modulating tissue; sensing physiological activity; sensing neural activity; sensing environmental conditions; sensing therapeutic outcomes; sensing delivered therapy parameters; and combinations thereof.

6. The medical apparatus according to any preceding claim, wherein the at least one extension (510) comprises an element selected from the group consisting of: a lead; an electrode; a conformable electrode; two sliding beams; a cuff electrode; an anchor element; an energy storage element; and a combination thereof.

7. The medical apparatus according to any preceding claim, wherein the implantable system further comprises an element (230) selected from the group consisting of: a tissue modulator; an actuator; an electromagnetic actuator; a sensor; and combinations thereof.

8. The medical apparatus according to any preceding claim, wherein the harvesting element is constructed and arranged to harvest energy from the patient, wherein the harvested energy comprises energy selected from the group consisting of: heat energy; motion energy; chemical energy; and combinations thereof.

9. The medical apparatus according to any preceding claim, wherein the implantable system comprises a power receiver (210) configured to receive energy selected from the group consisting of: radiofrequency energy; ultrasound energy; and combinations thereof.

**10.** The medical apparatus according to any preceding claim, wherein the implantable system further comprises an energy storage element selected from the group consisting of:
a battery; a capacitor; and combinations thereof.

**11.** The medical apparatus according to any preceding claim, wherein the transition of the expanded state comprises a physical change selected from the group consisting of:
unfolding; unrolling; unfurling; and combinations thereof.

**12.** The medical apparatus according to any preceding claim, wherein the at least one extension (510) comprises an inflatable portion constructed and arranged to expand when filled with fluid selected from the group of: a gas; a liquid; a gel; and combinations thereof.

**13.** The medical apparatus according to any preceding claim, wherein the at least one extension comprises an anchor element (800) selected from the group consisting of: threads; arrow-shaped element; harpoon-shaped element; and combinations thereof.

**14.** The medical apparatus according to any preceding claim, wherein the at least one extension (510) is constructed and arranged to detach from the body portion.

**15.** The medical apparatus according to any preceding claim, wherein the introducer (300) comprises a flexible cannula and a rigid cannula, and wherein the flexible cannula is constructed and arranged to slidingly receive the rigid cannula.


**Patentansprüche**

**1.** Medizinische Vorrichtung zum Herstellen einer Schnittstelleverbindung mit Gewebe eines Patienten, umfassend:

ein implantierbares System, umfassend einen Körperabschnitt (310) zum Implantieren in einem Patienten,
ein Energiegewinnungselement oder eine Antenne und Energiegewinnungsschaltungen, wobei das Energiegewinnungselement oder die Antenne und die Energiegewinnungsschaltungen in Form einer Erweiterung (510) ausgeführt sind, die an dem Körperabschnitt angebracht ist;
ein externes System (100), das während des Betriebs wenigstens eines von Leistung oder Daten transkutan an das implantierbare System überträgt; und
einen Inserter (300) zum Einbringen des Körperabschnitts (310) und der wenigstens einen Erweiterung (510) in den Patienten, wobei das implantierbare System dafür konstruiert und angeordnet ist, durch den Inserter bereitgestellt zu werden; und
wobei die Erweiterung (510) dafür ausgelegt ist, von einem von einem verdichteten Zustand in einen erweiterten Zustand überzugehen, um einen Radarquerschnitt des Energiegewinnungselements oder der Antenne zu vergrößern, um die Leistungsmenge, die das Energiegewinnungselement oder die Antenne ernten kann, zu erhöhen.

**2.** Medizinische Vorrichtung gemäß Anspruch 1, wobei die medizinische Vorrichtung dafür ausgelegt ist, eine physiologische Reaktion in Gewebe zu induzieren.

**3.** Medizinische Vorrichtung gemäß Anspruch 1 oder 2, wobei die medizinische Vorrichtung dafür ausgelegt ist, Gewebeaktivität zu überwachen.

**4.** Medizinische Vorrichtung gemäß einem der vorstehenden Ansprüche, wobei das externe System (100) dafür ausgelegt ist, sowohl Leistung als auch Daten während des Betriebs transkutan an das implantierbare System zu übertragen.

**5.** Medizinische Vorrichtung gemäß einem der vorstehenden Ansprüche, wobei das implantierbare System eine Funktion durchführt, die aus der Gruppe ausgewählt ist, bestehend aus: Bereitstellen von Energie für Gewebe; Bereitstellen einer Therapie für Gewebe; Bereitstellen eines pharmazeutischen Wirkstoffs für Gewebe; Modulieren von Gewebe; Erkennen physiologischer Aktivität; Erkennen neuraler Aktivität; Erkennen von Umgebungsbedingungen; Erkennen therapeutischer Ergebnisse; Erkennen bereitgestellter Therapieparameter; und Kombinationen davon.

**6.** Medizinische Vorrichtung gemäß einem der vorstehenden Ansprüche, wobei die wenigstens eine Erweiterung (510) ein Element umfasst, das aus der Gruppe ausgewählt ist, bestehend aus: einem Leiter; einer Elektrode; einer anpassungsfähigen Elektrode; zwei Schiebebalken; einer Manschettenelektrode; einem Ankerelement; einem Energiespeicherelement; und einer Kombination davon.

**7.** Medizinische Vorrichtung gemäß einem der vorstehenden Ansprüche, wobei das implantierbare System ferner ein Element (230) umfasst, das aus der Gruppe ausgewählt ist, bestehend aus: einem Gewebemodulator; einem Stellglied; einem elektromagnetischen Stellglied; einem Sensor; und Kombinationen davon.

**8.** Medizinische Vorrichtung gemäß einem der vorstehenden Ansprüche, wobei das Energiegewinnungselement dafür konstruiert und angeordnet ist, Energie von dem Patienten zu ernten, wobei die geerntete Energie Energie umfasst, die aus der Gruppe ausgewählt ist, bestehend aus: Hitzeenergie; Bewegungsenergie; chemische Energie; und Kombinationen davon.

**9.** Medizinische Vorrichtung gemäß einem der vorstehenden Ansprüche, wobei das implantierbare System einen Leistungsempfänger (210) umfasst, der dafür ausgelegt ist, Energie zu empfangen, die aus der Gruppe ausgewählt ist, bestehend aus: Funkfrequenzenergie; Ultraschallenergie; und Kombinationen davon.

**10.** Medizinische Vorrichtung gemäß einem der vorstehenden Ansprüche, wobei das implantierbare System ferner ein Energiespeicherelement umfasst, das aus der Gruppe ausgewählt ist, bestehend aus: einer Batterie; einem Kondensator; und Kombinationen davon.

**11.** Medizinische Vorrichtung gemäß einem der vorstehenden Ansprüche, wobei der Übergang des erweiterten Zustands einen physischen Wechsel umfasst, der aus der Gruppe ausgewählt ist, bestehend aus: Entfalten; Abrollen; Aufspannen; und Kombinationen davon.

**12.** Medizinische Vorrichtung gemäß einem der vorstehenden Ansprüche, wobei die wenigstens eine Erweiterung (510) einen aufblasbaren Abschnitt umfasst, der dafür konstruiert und angeordnet ist, sich auszudehnen, wenn er mit einem Fluid gefüllt wird, das aus der Gruppe ausgewählt ist, bestehend aus: einem Gas; einer Flüssigkeit; einem Gel; und Kombinationen davon.

**13.** Medizinische Vorrichtung gemäß einem der vorstehenden Ansprüche, wobei die wenigstens eine Erweiterung ein Ankerelement (800) umfasst, das aus der Gruppe ausgewählt ist, bestehend aus: Fäden bzw. Strängen; einem pfeilförmigen Element; einem harpunenförmigen Element; und Kombinationen davon.

**14.** Medizinische Vorrichtung gemäß einem der vorstehenden Ansprüche, wobei die wenigstens eine Erweiterung (510) dafür konstruiert und angeordnet ist, von dem Körperabschnitt gelöst zu werden.

**15.** Medizinische Vorrichtung gemäß einem der vorstehenden Ansprüche, wobei der Inserter (300) eine flexible Kanüle und eine starre Kanüle umfasst und wobei die flexible Kanüle dafür konstruiert und angeordnet ist, die starre Kanüle gleitend aufzunehmen.

men.

## Revendications

**1.** Appareil médical destiné à s'interfacer avec un tissus d'un patient comprenant :

un système implantable comprenant :

une partie corps (310) destinée à être implantée à l'intérieur d'un patient, un élément ou une antenne de collecte d'énergie, et un circuit de collecte d'énergie, l'élément ou l'antenne de collecte d'énergie et le circuit de collecte d'énergie se présentant sous la forme d'une extension (510) fixée à la partie corps ;

un système externe (100) qui transmet de manière transcutanée au moins une énergie ou des données au système implantable pendant son fonctionnement ; et un introducteur (300) pour introduire la partie corps (310) et l'au moins une extension (510) dans le patient ; le système implantable étant construit et agencé pour être posé par l'introducteur ; et l'extension (510) étant configurée pour passer d'un état compacté à un état étendu afin qu'une section transversale radar de l'élément ou de l'antenne de collecte d'énergie augmente la quantité d'énergie que ledit élément ou ladite antenne de collecte d'énergie peut collecter.

**2.** Appareil médical selon la revendication 1, l'appareil médical étant configuré pour induire une réponse physiologique dans un tissu.

**3.** Appareil médical selon la revendication 1 ou 2, l'appareil médical étant configuré pour surveiller l'activité tissulaire.

**4.** Appareil médical selon l'une quelconque des revendications précédentes, le système externe (100) étant configuré pour transmettre de manière transcutanée de l'énergie et des données au système implantable pendant le fonctionnement.

**5.** Appareil médical selon l'une quelconque des revendications précédentes, le système implantable réalisant une fonction sélectionnée dans le groupe constitué par : la fourniture d'énergie au tissu ; l'administration d'une thérapie au tissu ; l'administration d'un agent pharmaceutique au tissu ; la modulation du tissu ; la détection d'une activité physiologique ; la détection d'une activité neuronale ; la détection de conditions environnementales ; la détection de résultats thérapeutiques ; la détection de paramètres de thérapie administrée ; et des combinaisons de celles-ci.

6. Appareil médical selon l'une quelconque des revendications précédentes, l'au moins une extension (510) comprenant un élément sélectionné dans le groupe constitué par : un fil ; une électrode ; une électrode adaptable ; deux faisceaux coulissants ; une électrode de brassard ; un élément d'ancrage ; un élément de stockage d'énergie ; et une combinaison de ceux-ci.

7. Appareil médical selon l'une quelconque des revendications précédentes, le système implantable comprenant en outre un élément (230) sélectionné dans le groupe constitué par : un modulateur tissulaire ; un actionneur ; un actionneur électromagnétique ; un capteur ; et des combinaisons de ceux-ci.

8. Appareil médical selon l'une quelconque des revendications précédentes, l'élément de collecte étant construit et agencé pour collecter l'énergie provenant du patient, l'énergie collectée comprenant l'énergie sélectionnée dans le groupe constitué par : une énergie thermique ; une énergie de mouvement ; une énergie chimique ; et des combinaisons de celles-ci.

9. Appareil médical selon l'une quelconque des revendications précédentes, le système implantable comprenant un récepteur d'énergie (210) configuré pour recevoir de l'énergie sélectionnée dans le groupe constitué par : une énergie radiofréquence ; une énergie ultrasonique ; et des combinaisons de celles-ci.

10. Appareil médical selon l'une quelconque des revendications précédentes, le système implantable comprenant en outre un élément de stockage d'énergie sélectionné dans le groupe constitué par : une batterie ; un condensateur ; et des combinaisons de ceux-ci.

11. Appareil médical selon l'une quelconque des revendications précédentes, la transition de l'état dilaté comprenant un changement physique sélectionné dans le groupe constitué par : le dépliement ; le déroulement ; le déploiement ; et des combinaisons de ceux-ci.

12. Appareil médical selon l'une quelconque des revendications précédentes, l'au moins une extension (510) comprenant une partie gonflable construite et agencée pour se dilater lorsqu'elle est remplie d'un fluide sélectionné dans le groupe constitué par : un gaz, un liquide, un gel, et des combinaisons de ceux-ci.

ci.

13. Appareil médical selon l'une quelconque des revendications précédentes, l'au moins une extension comprenant un élément d'ancrage (800) sélectionné dans le groupe comprenant : des fils ; un élément en forme de flèche ; un élément en forme de harpon ; et des combinaisons de ceux-ci.

14. Appareil médical selon l'une quelconque des revendications précédentes, l'au moins une extension (510) étant construite et agencée pour se détacher de la partie corps.

15. Appareil médical selon l'une quelconque des revendications précédentes, l'introducteur (300) comprenant une canule souple et une canule rigide, et la canule souple étant construite et agencée pour recevoir de manière coulissante la canule rigide.

FIG. 1

FIG. 2

300    310    320

**FIG. 3**

310    410    320    420

**FIG. 4**

530

520

510

310

**FIG. 5**

620

610

310

**FIG. 6a**

FIG. 6b

FIG. 6c

**FIG. 7**

**FIG. 8**

**FIG. 9**

**FIG. 10**

**FIG. 11**

**FIG. 12**

1200

1220

1110

1120

1210

**FIG. 13**

1310

1300

**FIG. 14**

1400

1410

1420

**FIG. 15**

1500

1510

**FIG. 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 73477212
- US 20130215979 A
- WO 2008066556 A
- US 2006149330 A
- WO 2013035092 A
- US 2005043765 A
- US 2006224225 A
- US 2009204170 A
- US 2011301670 A
- WO 2007068284 A
- EP 2155330 A
- US 2010249888 A